# EUROPEAN PATENT APPLICATION

(11) **EP 2 921 145 A1**
(43) Date of publication of application: **23.09.2015**
(21) Application number: 14161376.0
(22) Date of filing: 24.03.2014
(51) Int. Cl.: A61F 5/01

(54) **Orthosis with four-axial mechanism stabilizing, especially knee joint orthosis**

(30) Priority: 22.03.2014 PL 40327214
(71) Applicant: MDH Sp. z o.o., 90-349 Poland (PL)
(72) Inventor: Szary, Jakub, 43-384 Jaworzec (PL)
(74) Representative: Rumpel, Alicja

(57) **Abstract**

The orthosis with a four-axial stabilizing mechanism, especially the knee joint orthosis, containing elements fastening the orthosis onto the limb which are connected permanently to the arms - the liners: upper (3) and lower (4) of the orthosis and containing at least one bolt (1) for fixing the locks (9), given that the bolt for fixing the locks blocks the extension of the orthosis, an extension-locking bolt (2), an upper plate (5), a lower plate (6), which connect the liners (3) and (4) and for each plate (5) and (6) there are two rotation axes, one for each liner (3) and (4), and the plate (5) or (6) is equipped with bolts (1) and (2)

## Description

The subject of the invention is an orthosis with a four-axial stabilizing mechanism, especially a knee joint orthosis

In the event of slight injuries the doctor taking care of the patient decides to completely immobilize the damaged limb due to the lack of tools stabilizing the limb in a satisfactory degree. Also the patients themselves often choose to put on the popular "cast" instead of old type orthoses equally restricting their movement. They contain mechanical elements being a type of hinges that relieve the joint that has been damaged. We know orthoses intended for stiffening joint connections in the directly post-traumatic and rehabilitation period.

Functional orthoses of the knee joint are used during the patient's returns to professional and sports activity after traumas and surgical treatments of the knee joint. They are designed to provide mechanical protection of the joint and prevent pathological movements (such as excessive tibiae translation) that could traumatize the joint or the nearby soft tissues. Functional orthoses are also designed to protect the joint against excessive encumbrance and prevent injuries in the vicinity of the knee joint. Usually functional orthoses also provide the possibility of restriction of the angular scope of movement to a safe range - proper for the indications of a specified therapeutic period. The most important element of a functional orthosis is thus the side stiffening along with a clock ensuring proper stabilization of the knee joint. In the case of a functional orthosis the structure as well as the functions of the clock are extremely important, since they are the ones that provide proper medical functions of the entire orthosis

In known solutions with monocentric hinges like, for example, the orthosis according to patent description US2011009786, the stiffening elements - the arms are connected in a rotational manner with a single-axial hinge. When the limb is bent as a result of operation of the hinges slightly different than the work of the joint the strips or cuffs keeping the orthosis on the limb experinece stress, which, in turn, may lead to an unwanted migration of the orthosis and improper stabilization of the joint. Therefore, they are used mostly to immobilize the limb or the cuffs keeping the orthosis on the limb, which, in turn, may lead to an unwanted migration of the orthosis and improper stabilization of the joint.

Mechanisms with two axes of rotation such as, for example, the one described in patent US7235058B2 where the elements orthosis the stiffening - the liners placed between two plates cooperate with each other by means of cut prongs rotating at the same time on two axes of the hinges, are also commonly known. Unfortunately in spite of the fact that this movement is similar to the one occurring in a knee joint, it does not take account of rolling and slipping motion.

Therefore, orthoses with multiaxial hinges making the movement of the orthosis similar to the movements of joints, e.g. the knee joint, have been gaining greater popularity.

An example of this type of mechanism is the solution used in patent EP1603497. This mechanism contains arms connected to two bases, given that one of the bases features a centrally placed element shaped in a way making it a lock of the maximum and the minimum opening or bending angle of the orthosis. The bases are not connected mutually to each other and the basis for which is not connected to the central element additionally contains a removable locks to adjust the maximum and the minimum opening or bending angle of the orthosis. As indicated by the clinical tests application of one central element causes overstiffening of the connection of the arms of the orthosis as a result of which the maximum bend or extension of the limb encounters sudden resistance, too strong in the case of fresh injuries.

A similar principle of operation is characterized by the solution disclosed in description WO2C033702, given that in this case the connection between the arms of the orthosis was made by using intermediate elements limiting the sudden overload accompanying the maximum inclinations of the arms of the orthosis whose contact with the additional element restricts the mobility of the joint. In this case the axes of the arms of the orthosis around which the rotation occurs move within the plates with guides cooperating with them. In this case after the maximum angle bend or extension is reached the hinge axis is shifted and the encumbrance on the joint is reduced. Unfortunately, the limiting of this sudden load may lead to an unacceptable, especially during the perioperiative period, inertia of the elements of the hinge and uncontrolled displacements of joint tissues.

For this reason, it was advisable to design a hinge of an rehabilitation orthosis that would ensure anatomical mobility of the joint without inducing displacements of the elements of the orthosis along the limb axis and unwanted shifts of the joint itself along with simultaneous elimination of the strains appearing when the maximum extension or bending angle is reached.

An orthosis with a four-axial stabilizing mechanism, especially a knee joint orthosis, contains elements fastening the orthosis onto the limb which are connected permanently to the arms - the liners: the upper and the lower liner of the orthosis and containing at least one bolt for fixing the locks, given that the lock-fixing bolt blocks the extension of the orthosis, an extension-locking bolt, an upper plate, a lower plate, which connect the liners and for each of the plates there are two rotation axes, one for each liner and a plate is equipped with bolts

The liners are connected indirectly with each other with at least one pair of plates - the upper and the lower, and for every connecting plate there are two rotation axes, one for each liner. One of the connecting plates is equipped with at least three bolts, at least one of which cooperates with the removable lock. Preferably, the bolts in which the connecting plate is equipped are connected with the plate in such a way that after the maximum extension or bending angle of the orthosis is reached they suppress the overload that follows. Preferably, the mandrels in which the plate is equipped have a layer structure and their external layer is flexible and preferably made of caoutchouc or rubber.

The rotation axes of the liner are rivets and the locks are equipped with a groove (notch) that makes it possible to fix the lock on the bolt as well as a threaded hole making it possible to restrain it by using a screw passing through the hole in the connecting plate. The lock restricting the full range of bending is established by contact of at least one bolt placed on the connecting plate the oblong element of the liner cooperating with it. The fixed bending or extension angle lock is established by simultaneous supporting the shaped liners against the edges of the locks, given that during the maximum extension the two bolts are based on a single liner and the maximum bend is limited by the contact of one of the bolts with the liner.

The connection of the liners as well as the connecting plates is made by using any known technology, preferably with rivets in such a way that during the shift of the angle between the liners simultaneous rotary movement as well as slide of the kinematic chain of the hinge (lock) of the orthosis occurs.

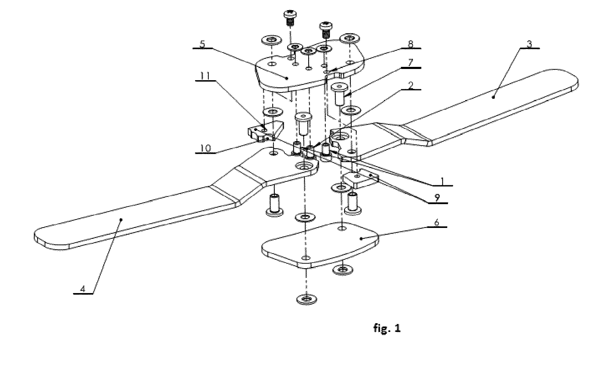

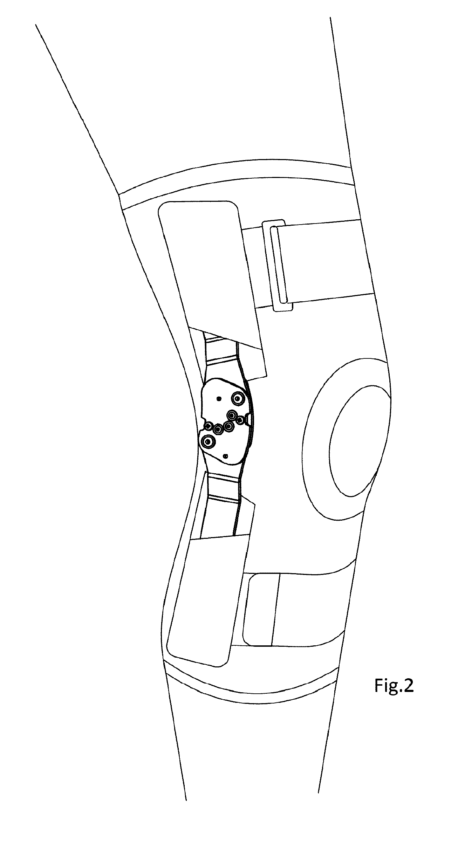

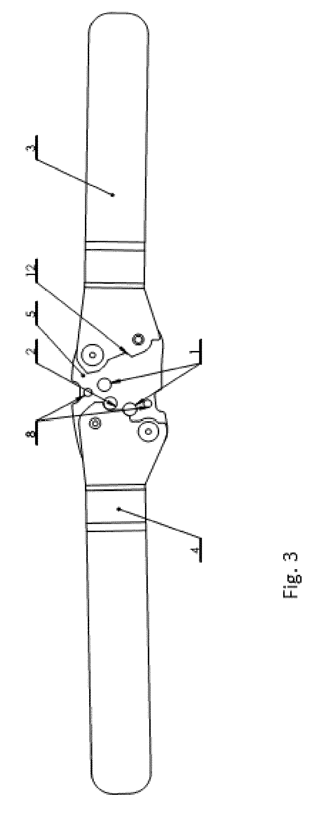

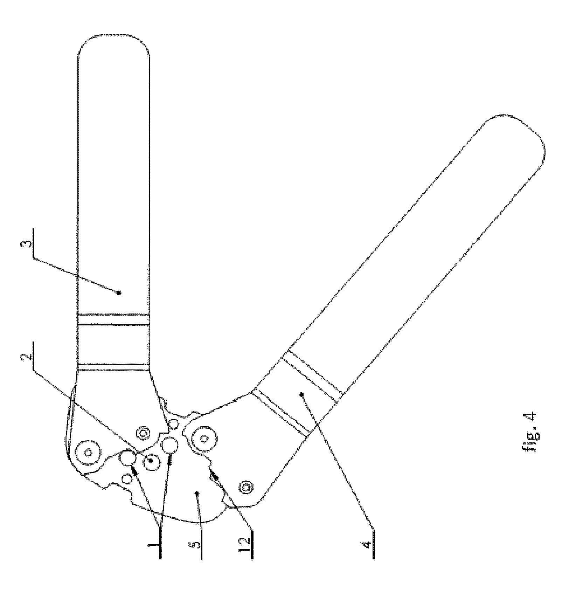

The orthosis with a four-axial stabilizing mechanism, especially the knee joint orthosis, according to the invention has been presented in the drawing, in which fig. 1 presents the elements of the four-axial mechanism stabilizing the orthosis according to the invention, fig. 2 presents the orthosis according to the invention in the working position, fig. 3 presents the mechanism of the orthosis according to the invention in the maximally extended position and fig. 4 presents the mechanism of the orthosis according to the invention in the maximum bending position.

### Example I

The orthosis with a four-axial stabilizing mechanism, especially the knee joint orthosis, according to the invention contains elements fastening the mechanism onto the limb (not shown in the drawing) which are connected permanently to the arms - the liners: upper 3 and lower 4 of the orthosis, bolt 1 to fasten locks 9, given that the bolt for fixing the locks blocks the extension of the orthosis, bolt 2 to locking the extension, upper plate 5, lower plate 6.

Liners 3 and 4 are connected indirectly with each other by a pair of plates - upper 3 and lower 4 and for every plate 5 and 6 there are two rotation axes, one for each liner 5 and 6. One of the plates, 3 or 4, is equipped with bolts 1 and 2, of which bolt 1 cooperates with the removable lock. The bolts which the connecting plate is equipped with are connected with the plate in such a way that after the maximum extension or bending angle of the orthosis is reached they suppress the overload that follows. Mandrels 1 and in 2 the plate is equipped with have a layer structure and their external layer is flexible.

The rotation axes of the liner are rivets 7, and locks 9 are equipped with groove 10 (notch) that makes it possible to fix the lock on bolt 1 as well as threaded hole 8 making it possible to restrain it by using a screw passing through the hole in the connecting plate. Lock 9 restricting the full range of bending is established by contact of bolt 1 placed on the connecting plate with the oblong element of liner 3 and 4 cooperating with it. The lock of the fixed bending or extension angle is established by simultaneous supporting the shaped liners against the edges of lock 9, given that during the maximum extension the two bolts are based on a single liner and the maximum bend is limited by the contact of one of the bolts with the liner.

The connection of the liners as well as the connecting plates is made by using any known technology, preferably with rivets 7 in such a way that during the shift of the angle between the liners simultaneous rotary movement as well as slide of the kinematic chain of the hinges, (lock) of the orthosis occurs.

## Claims

1. The orthosis with a four-axial stabilizing mechanism, especially the knee joint orthosis, containing elements fastening the orthosis onto the limb which are connected permanently to the arms - the liners: upper (3) and lower (4) of the mechanism is significant by the fact that it contains at least one bolt (1) for fixing the locks (9), given that the bolt for fixing the locks blocks the extension of the mechanism, an extension-locking bolt (2), an upper plate (5), a lower plate (6), which connect the liners (3) and (4) and for each plate (5) and (6) there are two rotation axes, one for each liner (3) and (4), and the plate (5) or (6) is equipped with bolts (1) and (2).

2. According to claim 1 the orthosis is significant by the fact that the bolt (1) cooperates with the removable lock (9).

3. According to claim 1 or 2 the orthosis is significant by the fact that the bolts which the connecting plate is equipped with are connected with the plate (5) or (6) in such a way that after the maximum extension or bending angle of the orthosis is reached they suppress the overload that follows.

4. According to claim 3 the orthosis is significant by the fact that bolts (1) and (2) in which the plate is equipped have a layer structure and their external layer is flexible.

5. According to claim 1 or 2, or 3 or 4 the orthosis is significant by the fact that the plates (5) and (6) contain the rotation axes of the liner made/produced via the plates (3) and (4) rivets (7) placed in the holes.

6. According to any of the previous claims the orthosis is significant by the fact that the bolts combined with the plate (5) or (6) in a way making them cooperate with the groove (10) of lock 9 and the plates (5) and (6) are equipped with a hole corresponding in the location to the threaded hole (8) of the lock (9),

7. According to claim 6 the orthosis is significant by the fact that the lock of the fixed bending or extension angle is established by simultaneous supporting the shaped liners (3) and (4) against the edges of the lock (9), given that during the maximum extension the two bolts (1) and (2) are based on a single liner and the maximum bend is limited by the contact of the bolt (1) with the liner.

8. According to any of the previous claims the orthosis is significant by the fact that the connection of the liners as well as the connecting plates made using rivets (7) in such a way that during the shift of the angle between the liners simultaneous rotary movement as well as slide of the kinematic chain of the hinge (lock) of the orthosis occurs.
